# EUROPEAN PATENT APPLICATION

(11) **EP 2 258 862 A1**
(43) Date of publication of application: **08.12.2010**
(21) Application number: 09712639.5
(22) Date of filing: 20.02.2009
(51) Int. Cl.: C12Q 1/68

(54) **CHIP FOR HPV GENOTYPING**

(30) Priority: 21.02.2008 KR 20080016007
(71) Applicant: Chabiotech Co., Ltd., Seoul 135-080 (KR); Biometrix Technology Inc., Kangwon-Do 200-161 (KR)
(72) Inventor: KO, Jung-Jae, Yongin-si Gyeonggi-do 446-916 (KR); CHUNG, Kwang-Hoe, Seongnam-si Gyeonggi-do 463-050 (KR); AN, Hee-Jung, Seoul 135-280 (KR); KIM, Tae-Sun, Chuncheon-si Gangwon-do 200-160 (KR); SONG, Keum-Soo, Chuncheon-si Gangwon-do 200-140 (KR); KIM, Hyung-Sub, Chuncheon-si Gangwon-do 200-160 (KR); EOUM, Woon-Yong, Chuncheon-si Gangwon-do 200-160 (KR)
(74) Representative: Winkler, Andreas Fritz Ernst
(86) International application number: PCT/KR2009/000823
(87) International publication number: WO 2009/104926

(57) **Abstract**

The present invention relates to a DNA chip for detecting or typing human papilloma virus (HPV), which comprises (a) a solid substrate of which the surface is coated with a calixarene derivative and (b) a probe hybridizable with the HPV nudeotide sequence which is fixed on the solid substrate. The prove comprises (i) a hybridization site for hybridizing the probe with the HPV nucleotide sequence and (ii) a fixation site containing at least two guanines. Since the DNA chip for HPV genotyping is configured in a strip, the application of the sample to be analyzed and analysis of the results are easy. In addition, the disclosed DNA chip can perform a quantitative analysis of HPV in the sample besides the HPV genotyping.

Sequence listing
<110>	BIOMETRIX TECHNOLOGY, Inc.
	CHA Biomed Co., Ltd.

<120>	Chips for Genotyping HPV

<150>	KR10-2008-0016007
<151>	2008-02-21

<160>	19

<170>	KopatentIn 1.71

<210>	1
<211>	28
<212>	DNA
<213>	Artificial Sequence

<220>
<223>	HPV type 16 specific probe


<400>	1
gggggggggt tcaaattatt ttcctaca	28


<210>	2
<211>	26
<212>	DNA
<213>	Artificial Sequence

<220>
<223>	HPV type 18 specific probe


<400>	2
gggggggggg tgtgtattct ccctct	26


<210>	3
<211>	27
<212>	DNA
<213>	Artificial Sequence

<220>
<223>	HPV type 6 specific probe


<400>	3
ggggggggga gtatatatgt taacacc	27


<210>	4
<211>	39
<212>	DNA
<213>	Artificial Sequence

<220>
<223>	HPV type 11 specific probe


<400>	4
gggggggggt ctgtagctac tagtatttat gtacataca	39


<210>	5
<211>	26
<212>	DNA
<213>	Artificial Sequence

<220>
<223>    beta-globin specific probe

<400>    5
   gggggggggc tgccctgtgg ggcaag                                          26

<210>    6
<211>    27
<212>    DNA
<213>    Artificial Sequence

<220>
<223>    HPV type DNA forward primer for PCR amplification

<400>    6
gatggtgata tggtagatac aggattt                                        27

<210>    7
<211>    29
<212>    DNA
<213>    Artificial Sequence

<220>
<223>    HPV type DNA reverse primer for PCR amplification

<400>    7
cctagtggct ctatggtaac ctctgacgc                                      29

<210>    8
<211>    29
<212>    DNA
<213>    Artificial Sequence

<220>
<223>    PC-1 type 1 probe

<400>    8
gggggggggt attttcctac acctagtgg                                      29

<210>    9
<211>    29
<212>    DNA
<213>    Artificial Sequence

<220>
<223>    PC-1 type 2 probe

<400>    9
gggggggggt attctccctc tcctagtgg                                      29

<210>    10
<211>    29
<212>    DNA
<213>    Artificial Sequence

<220>
<223>    PC-2 type 1 probe

<400>    10
ggggggggt atgttaacac ccctagtgg                                    29

<210>    11
<211>    29
<212>    DNA
<213>    Artificial Sequence

<220>
<223>    PC-2 type 2 probe

<400>    11
ggggggggt atgtacatac acctagtgg                                    29

<210>    12
<211>    33
<212>    DNA
<213>    Artificial Sequence

<220>
<223>    NC probe

<400>    12
ggggggggga aagctgctgc tcgtcgtcgt cgt                               33

<210>    13
<211>    39
<212>    DNA
<213>    Artificial Sequence

<220>
<223>    HC probe

<400>    13
gggggggggt ttacacctag tggctctatg gtgtcctct                         39

<210>    14
<211>    20
<212>    DNA
<213>    Artificial Sequence

<220>
<223>    HPV type DNA forward primer for target DNA

<400>    14
caacttcatc cacgttcacc                                             20

<210>    15
<211>    20
<212>    DNA
<213>    Artificial Sequence

<220>
<223>    HPV type DNA reverse primer for target DNA

<400>    15

ggtgaacgtg gatgaagttg 20

<210> 16
<211> 27
<212> DNA
<213> Artificial Sequence

<220>
<223> HPV forward primer

<400> 16
gatggtgata tggtagatac aggattt 27

<210> 17
<211> 29
<212> DNA
<213> Artificial Sequence

<220>
<223> HPV reverse primer

<400> 17
cctagtggct ctatggtaac ctctgacgc 29

<210> 18
<211> 20
<212> DNA
<213> Artificial Sequence

<220>
<223> Beta-G forward primer

<400> 18
caacttcatc cacgttcacc 20

<210> 19
<211> 20
<212> DNA
<213> Artificial Sequence

<220>
<223> Beta-G reverse primer

<400> 19
ggtgaacgtg gatgaagttg 20

## Description

### BACKGROUND OF THE INVENTION

### FIELD OF THE INVENTION

The present invention relates to a DNA chip for detecting or genotyping HPV.

### DESCRIPTION OF THE RELATED ART

### SUMMARY OF THE INVENTION

Since Affymetrix Co. U.S.A reported the technology of preparing a DNA chip by selectively binding bases on a solid substrate using surface photosynthesis technology utilizing photomask, various techniques for preparing gene chips were developed, such as an aldehyde chip, fixing probe gene on various solid substrate surface by chemical bonds. Biometrix Technology Inc. (BMT) of Korea has developed various methods of preparing DNA chips by converting the solid surface into monolayer of macromolecues, which can simultaneously immobilize oligo-DNAs containing consecutive guanines on a solid substrate.

Various DNA chips have been developed worldwide for genotyping diseases such as human papilloma viruses (HPV) or tuberculosis.

Using DNA chip for genotyping has the two following limitations.

First, DNA chips for genotyping is covered with a PCR product amplified using signal detection reagents, especially fluorescent labeled primers. The fluorescent intensity is detected when a probe affixed on the chip and a perfect matching gene probe hybridizes, thus enabling the genotyping of the virus. Problems may occur when false positive fluorescent signals are detected by genes binding to a different genotyping probe by nonspecific binding. The washing step following the hybridization is required to eliminate these nonspecific bindings. Reducing the amount of nonspecifically bound DNA through the washing step is highly dependent on the skill of the researcher; therefore it is hard to reduce false positive results in many of the newly developed HPV genotyping chips. To achieve reproducible data, DNA genotyping technology using a strip should be used, where hydrodynamically identical solvents injected will move at a constant speed through the uniform space, achieving identical washing.

Second, the genotyping of HPV virus by fluorescent labeled PCR amplification product use scanners that cost several tens of thousands of dollars. These expensive scanners are too costly for the researcher to buy and use for their experiment, therefore a cheaper analyzer that cost several thousands of dollars with simple structure is needed. Therefore, an optimal genotyping DNA chip for this scanner will be in the form of a membrane strip. The DNA strip where probe DNA for genotyping is applied on the strip as a linear form, immobilized, then the selective hybridization reaction is detected with a strip reader after flowing the hybridization solution would be in high demand.

Throughout this application, various patents and publications are referenced and citations are provided in parentheses. The disclosure of these patents and publications in their entities are hereby incorporated by references into this application in order to more fully describe this invention and the state of the art to which this invention pertains.

### DETAILED DESCRIPTION OF THE INVENTION

The present inventors have conducted intensive researches to develop a method for detecting and genotyping HPV, especially a rapid kit method using strip type solid substrate. As a result, the inventors have discovered that coating the solid surface with calixarene derivatives with special chemical structures then immobilizing with the oligonucleotide was a simple and accurate method for detecting and genotyping HPV, and thus completed the present invention.

Accordingly, it is an object of this invention to provide a DNA chip for determining and genotyping HPV.

Other objects and advantages of the present invention will become apparent from the detailed description to follow and together with the appended claims and drawings.

In one aspect of this invention, there is provided a DNA chip for detecting or genotyping HPV, which comprises (a) a calixarene derivative coated on the solid substrate surface; and (b) a probe hybridizable with a nucleotide sequence of HPV (human papilloma virus) immobilized on the surface of the solid substrate, wherein the probe comprises (i) a hybridizing portion hybridizable with the nudeotide sequence of HPV and (ii) an immobilization portion having at least two guanines.

The present inventors have conducted intensive researches to develop a method for detecting and genotyping HPV, especially a rapid kit method using strip type solid substrate. As a result inventors have discovered that coating the solid surface with calixarene derivatives with special chemical structures then immobilizing with the oligonucleotide was a simple and accurate method for detecting and genotyping HPV.

According to a preferred embodiment, the calixarene derivative is represented by the following Formula 1, Formula 2 or Formula 3: 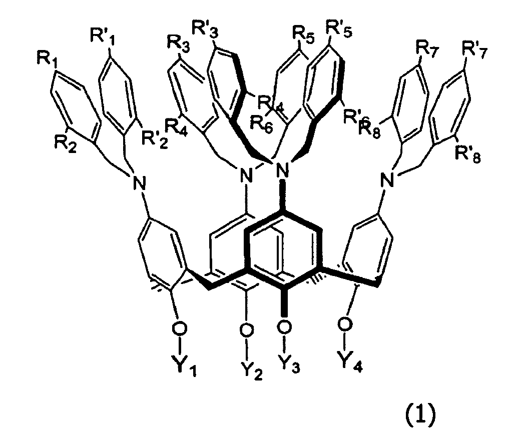 wherein, R₁, R₂, R₃, R₄, R₅, R₆, R₇, R₈, R'₁ ,R'₂ ,R'₃ ,R'₄ ,R'₅ ,R' ₆ ,R '₇ and R'₈ independently represent -H, -CH₃, -C₂H₅, -C₃H₇, -OCH₃, - Cl, -C6H₅, -OH, -OCH₂CH₃, -Br, -CF₃, - OCH₂C₆H₅ -CC₆H₅, -OC₆H₄CH₃, -OC₆H4C(CH₃)₃, -OC₆H₄CF₃, -C₆H₄Cl, -OCOCH₃ , -NHCOCH₃ , - CONHCH₃ , -CN, -COOH or -COOR; R in -COOR is -CH₃ or -C₂H₅; Y₁, Y₂, Y₃ and Y₄ independently represent -H, - (CH₂)ₙ -(CH=O, -(CH₂)ₙ-SH, -(CH₂CH₂O)ₘ-CH₂CH₂-CH=O, - (CH₂CH₂O)ₘ-CH₂CH₂-SH, -(CH₂)ₘ-C₆H₄-(CH₂)_{c}-Z or -CO-(CH₂)ₘ₋₁-C₆H₄-(CH₂)_{c}-Z; Z represents - SH, -CHO, -COOH or -NH₂; -C₆H₄- and C₆H₅represent a phenyl group; n is an integer of 2-15, m is an integer of 1-10, and c is an integer of 0-10; 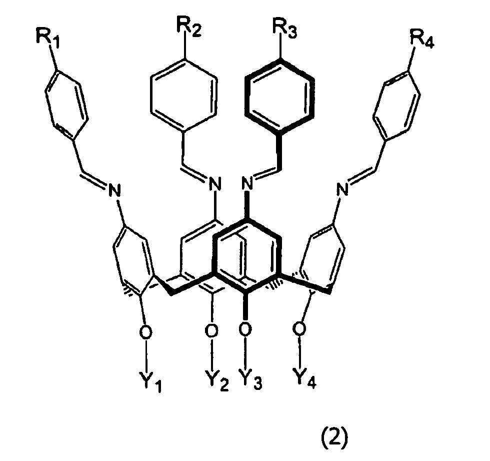 wherein, R₁, R₂, R₃ and R₄ independently represents -H, -CH₃, -C₂H₅, -C₃H₇, -OCH₃, - Cl, -C₆H₅, -OH, -OCH₂CH₃, -Br, -CF₃ , -OCH₂C₆H₅ -OC₆H₅, -OC₆H₄CH₃, -OC₆H₄C(CH₃)₃, -OC₆H₄CF₃, - C₆H₄Cl, -OCOCH₃ , -NHCOCH₃ , -CONHCH₃ , -CN, -COOH or -COOR; R in -COOR is -CH₃ or - C₂H₅; Y₁, Y₂, Y₃ and Y₄ independently represent -H, - (CH₂)ₙ -CH=O, -(CH₂)ₙ-SH, -(CH₂CH₂O)ₘ-CH₂CH₂-CH=O, -(CH₂CH₂O)ₘ-CH₂CH₂-SH, -(CH₂)ₘ-C₆H₄-(CH₂)_{c}-Z or -CO-(CH₂)ₘ₋₁-C₆H₄-(CH₂)_{c}-Z ; Z represents -SH, -CHO, -COOH or -NH₂; -C₆H₄- and C₆H₅ represent phenyl groups; n is an integer of 2-15, m is an integer of 1-10, and c is an integer of 0-10; 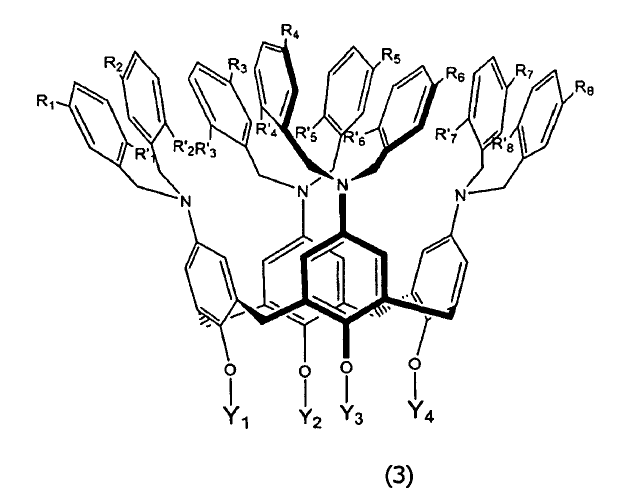 wherein, R₁, R₂, R₃, R₄, R₅, R ₆, R₇, R₈, R'₁ ,R'₂ ,R'₃ ,R' ₄ ,R' ₅ ,R' ₆ ,R '₇ and R'8 independents represent -H, -CH₃, -C₂H₅, -C₃H₇, -OCH₃, - Cl, -C₆H₅ , -OH, -OCH₂CH₃, -Br, -CF₃ , -OCH₂C₆H₅ -OC₆H₅, -OC₆H₄CH₃, -OC₆H₄C(CH₃)₃, -OC₆H₄CF₃, -OC₆H₄Cl, -OCOCH₃, -NHCOCH₃, - CONHCH₃ , -CN, -COOH or -COOR; -COLOR; R in -COOR is -CH₃ or -C₂H₅; Y₁, Y₂, Y₃ and Y₄ independently represent -H, -(CH₂)ₙ-CH=O, -(CH₂)ₙ-SH, -(CH₂CH₂O)ₘ-CH₂CH₂-CH=O, - (CH₂CH₂O)ₘ-CH₂CH₂-SH, -(CH₂)ₘ-C₆H₄-(CH₂)_{c}-Z or -CO-(CH₂)ₘ₋₁-C₆H₄-(CH₂)_{c}-Z ; Z represents - SH, -CHO, -COOH or -NH₂; -C₆H₄- and C₅H₅ are phenyl groups; n is an integer of 2-15, m is an integer of 1-10, and c is an integer of 0-10; with an exception of the following compound: all of R₁, R₂, R₃, R₄, R₅, R₆, R₇, R₈, R'₁ ,R'₂ ,R'₃ ,R' ₄ ,R' ₅ ,R' ₆ ,R'₇ and R'₈ are -H.

One characteristics of this invention is to utilize a solid substrate of which surface is coated with calixarene derivatives represented by Formula 1, Formula 2 or Formula 3. The calixarene derivatives are developed by the inventors and described in details in Korean Pat. No. 698763 and Korean Pat. No. 748082, which are incorporated herein by reference in their entity. Formula 1 and 3 are aminocalixarene derivatives and Formula 2 is an iminecalixarene derivative.

The calixarene derivatives represented by Formula 1, Formula 2 or Formula 3 form a self-assembled monolayer (SAM) when coated on the solid substrate.

Another characteristic of this invention is that the probe used in this invention comprises (i) a hybridizing portion hybridizable with the nudeotide sequence of HPV and (ii) an immobilization portion having at least two guanines.

The term "probe" used herein refers to native or modified monomer, or linear oligomers with linkages, capable of specifically binding to a target nucleotide sequence, and is naturally-occurring or may be artificially synthesized. The probe is preferably in the form of a single strand, more preferably an oligodeoxyribonucleotide. The probe may indude naturally occurring dNMPs (dAMP, dGMP, dCMP and dTMP), nudeotide analogues or derivatives. The probe is a backbone modified nucleotide, *e.g*., peptide nucleic add (PNA) (M. Egholm et al., Nature, 365:566-568 (1993)), phosphorothioate DNA, phosphorodithioate DNA, phosphoroamidate DNA, amide-linked DNA, MMI-linked DNA, 2'-O-methyl RNA, alpha-DNA and methylphosphonate DNA, sugar modified nucleotide, *e.g*., 2'-O-methp RNA, 2'-fluoro RNA, 2'-amino RNA, 2'-O-alky) DNA, 2'-Q-aryl DNA, 2'-O-alkinyl DNA, hexose DNA, pyranosyl RNA and anhydrohexitol DNA, and base modified nucleotide, e.g., C-5 substituted pyrimidine (substituents indude fluoro-, bromo-, chloro-, iodo-, methyl-, ethyl-, vinyl-, formyl-, ethynyl-, propynyl-, alkynyl- thiazolyl-, imidazolyl- and pyridyl-), C-7 substituted 7-deazapurine (substituents indude fluoro-, bromo-, chloro-, iodo-, methyl-, ethyl-, vinyl-, fiormyl-, alkynyl-alkenyl-, thiazolyl-, imidazolyl- and pyridyl-), inosine and diaminopurine.

The probe used in this invention comprises two portions. The "hybridization portion" is complementary to the HPV target nudeotide sequence and hybridizes to the nudeotide sequences of HPV "Immobilization portion" is the region that immobilizes to the calixarene monolayer that is coated on the solid substrate and has at least two guanines. The guanines in the immobilization portion help the probe immobilize on the calixarene monolayer through multiple molecular interactions with 4 nitrogen atoms in calixarene derivatives.

It is preferable that two or more guanines are consecutive bases. According to the preferred embodiment, at least two guanines in the immobilization portion of the probe are located at 5'- or 3'- end, and more preferably, 5'- end.

The term "guanine" with conjunction with probes refers to a nudeotide with a guanine base.

According to the preferred embodiment, the probe hybridizable to the HPV nudeotide sequence comprises 7-15 guanines at their 5'- or 3'- end, more preferably, comprising 7-10 guanines.

The solid substrate used in the present chip is not particularly limited, but it may indude any conventional solid substrate. Preferably, the solid substrate is glass, glass fiber, metal (e.g., gold, gold-copper alloy, aluminum), metal oxide, ceramic, quartz, silicon, semi conductor, Si/SiO₂, wafer, germanium, gallium arsenide, carbon, carbon nanotube, polymer (e.g., polystyrene, polyethylene, polypropylene, polyacrylamide), sepharose, agarose or colloid.

This invention is developed to detect and identify the genotype of HPV by a rapid kit using strip type solid substrates. Therefore, it is preferable to use a glass fiber substrate when constructing a rapid kit in this invention. The sample in a liquid form flows or moves in the solid substrate of the rapid kit. The rapid kit uses a method generally called a chromatography analysis. The kit of this invention is advantageous for constructing rapid kits. In glass fibers, the solution (*e.g*., PCR amplified gene sample) applied at one end flows against the force of gravity or moves via capillary action similar to general fibers and membranes. By using the above-mentioned characteristics of the glass fiber in this invention, HPV PCR amplified products with labels (*e.g*., fluorescent molecules) flow and hybridize with the immobilized probes for HPV genotyping, thereby ensuring identification of HPV genotypes.

This invention is very useful not only for detecting HPV but also for determining the HPV genotypes. For HPV genotyping, the DNA chip of this invention has probes affixed on the solid substrate that are specifically hybridizable with at least two HPV type nucleotide sequences selected from the group consisting of HPV type 16, 18, 31, 33, 35, 39, 45, 51, 52, 56, 58, 59 and 68.

The genotype of HPV has various HPV types which are known to about 100 types of HPV, up to date. Of them, HPV types 16, 18, 31, 33, 35, 39, 45, 51, 52, 56, 58, 59 and 68 are known as high risk types and HPV types 6, 11, 43, 43 and 44 as low risk types.

The detailed examples of the HPV genotyping probes are described in SEQ ID NOs:1-4.

According to the preferred embodiment, the DNA chip in the present invention further comprises a probe that is hybridized with a housekeeping gene in human cells. The term "housekeeping gene" used herein refers to a constitutively expressed gene that is required for the maintenance of the basal cellular function (Watson et al., Molecular Biology of the Gene, Vol. 1, 1965).

The probe hybridized with the housekeeping gene is used to normalize the final signal from the chip of this invention, enabling quantification of HPV genes. As demonstrated in Example 5, the levels of HPV genotypes may be quantified by analyzing the ratio of the hybridization signals (*e.g*., fluorescent intensity) from high and low levels of housekeeping gene (beta-globin gene) and HPV genotypes.

The probe used in the chip has nudeotide sequences that can hybridize with the housekeeping gene, preferably GAPDH (glyceraldehydes phosphate dehydrogenase) gene, beta-actin gene, PGK1 (phosphoglycerate kinase 1) gene, beta-globin gene or 28S RNA gene.

It is preferable to construct the chip of the present invention in the form of a rapid kit method having a strip type solid substrate. According to the preferred embodiment, the DNA chip of the present invention has a membrane strip type. At least two probes are immobilized on specific areas on the membrane strip and the DNA sample for analysis is applied at one end of the membrane and allowed to flow on the membrane strip surface.

More preferably, the DNA chip of the present invention comprises (i) a glass fiber membrane strip as solid substrates, (ii) at least two probes hybridizable with at least two HPV type nucleotide sequences selected from the group consisting of HPV type 16, 18, 31, 33, 35, 39, 45, 51, 52, 56, 58, 59 and 68; the probe is immobilized on specific areas on the membrane strip, and (iii) a probe hybridizable with a housekeeping gene in human cells.

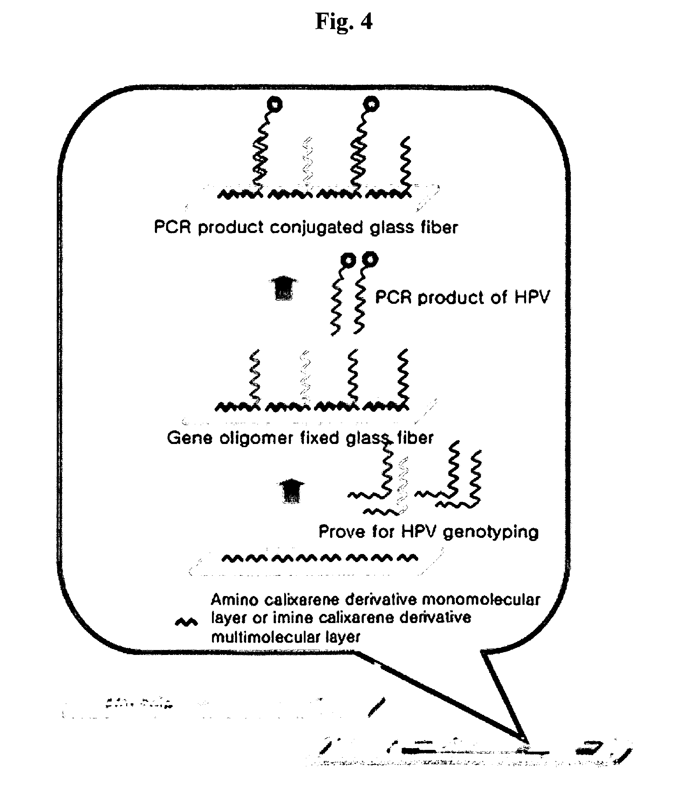

The present invention overcomes shortcomings associated with conventional HPV genotyping DNA chips. According to this invention, the probes hybridizable with the HPV genotype are linearly immobilized on the glass strip as shown in Fig. 4. This strip typed genotyping chip enables the determination of the HPV genotype through the hybridization reaction between the DNA samples (*e.g*., PCR amplification product) flowing on the strip surface and the gene specific probes.

According to the preferred embodiment, the probe hybridizable with the solid surface or DNA samples to be analyzed is labeled with molecules generating detectable signal. For instance, chemical label (*e.g*., biotin), radio label (*e.g*., 1125 and C¹⁴), fluorescent label (*e.g*., fluorescein, phycoeritrin, rhodamin, lissamin, TAMRA, Cy5, Cy3, HEX, TET, Dabsyl and FAM), illuminant label, chemiluminescent label, FRET (fluorescence resonance energy transfer) label or heavy metal (*e.g*., gold). Labeling with signal molecules may be performed in accordance with any one of the conventional methods, *e.g*., nick translation method, Multiprime DNA labeling method (Multiprime DNA labeling systems booklet, "Amersham" (1989)) and kination method (Maxam & Gilbert, Methods in Enzymology, 65:499 (1986)).

The DNA chip ensures the detection of HPV and quantification of HPV through the hybridization reaction between the probe and the DNA sample to be analyzed.

The optimal condition for the hybridization can be decided according to the methods described in the following references, Joseph Sambrook et al., Molecular Coning, A Laboratory Manual, Cold Spring Harbor Laboratory Press, Cold Spring Harbor, N.Y (2001) and Haymes, B.D., et al., Nucleic Add Hybridization, A practical Approach, IRL Press, Washington, D.C. (1985). A stringent condition for hybridization can be established by controlling the temperature, ionic strength (buffer concentration) and the amount of compounds, such as organic solvents, in the buffers system. The stringent condition can also be modified according to the hybridizing sequences.

For example, highly stringent condition in the stringent condition is hybridization at 65°C in hybridization buffer of 0.5 M NaHPO₄, 7% SDS (Sodium dodecyl sulfate), 1 mM EDTA, then washed at 68°C with wash buffer of 0.1 × SSC (standard saline citrate)/0.1% SDS. The chip is washed again at 48°C with wash buffer of 6 × SSC/0.05% sodium pyrophosphate. Low stringent condition is washing at 42°C with 0.2 × SSC/0.1% SDS wash buffer.

According to the preferred embodiment, the DNA chip further comprises a hybridization control probe hybridizable with a primers used to amplify the HPV nucleotide sequences of HPV in an analysis sample.

Generally, the DNA applied on the DNA chip is isolated from the sample and amplified by PCR (polymerase chain reaction). That is, the PCR amplification product isolated from the DNA of the analysis sample is applied on the DNA chip. The DNA chip in this invention further comprises hybridization control probe hybridizable with the primers used to amplify the HPV nudeotide sequences in the analysis sample (*e.g*., cells from uterine cervix). The hybridization control probes are hybridizable with the free probes remaining in the PCR amplification products. The signal generated from the hybridization control probe dramatically reduces the possibility of false signals by the DNA chip. Where a hybridization signal is detected from the hybridization control probe but not from the HPV specific probes, the sample is determined not to have any HPV. However, if no signal is detected from both the hybridization control probe and HPV specific probe, this would be considered as a false negative.

Preferably, the hybridization control probe is a probe which binds to a reverse primer used for HPV nucleotide sequence amplification, more preferably, a probe hybridizable with a fluorescent labeled reverse primer used for HPV nucleotide sequence amplification. The detailed description of hybridization control primers used in this invention is shown in Table 2.

According to the preferred embodiment, the DNA chip further comprises positive control probe hybridizable with the HPV type nonspecific nudeotide sequences. The detailed description of positive control primers used in this invention is shown in Table 2.

According to the preferred embodiment, the DNA chip further comprises negative control probe that does not hybridize to the HPV nucleotide sequences. The detailed description of negative control primers used in this invention is shown in Table 2.

The features and advantages of the present invention will be summarized as follows:
(i) The DNA chip for genotyping HPV in this invention is highly useful for detecting and genotyping HPV.
(ii) The DNA chip for genotyping HPV in this invention has the advantage to be built as a strip form.
(iii) The DNA chip in this invention, wherein built as a strip form, is convenient for applying samples and analyzing the test results.
(iv) This invention will provide quantitative analysis of HPV in the samples in addition to determination of HPV genotypes.

### DETAILED DESCRIPTION OF THE DRAWINGS

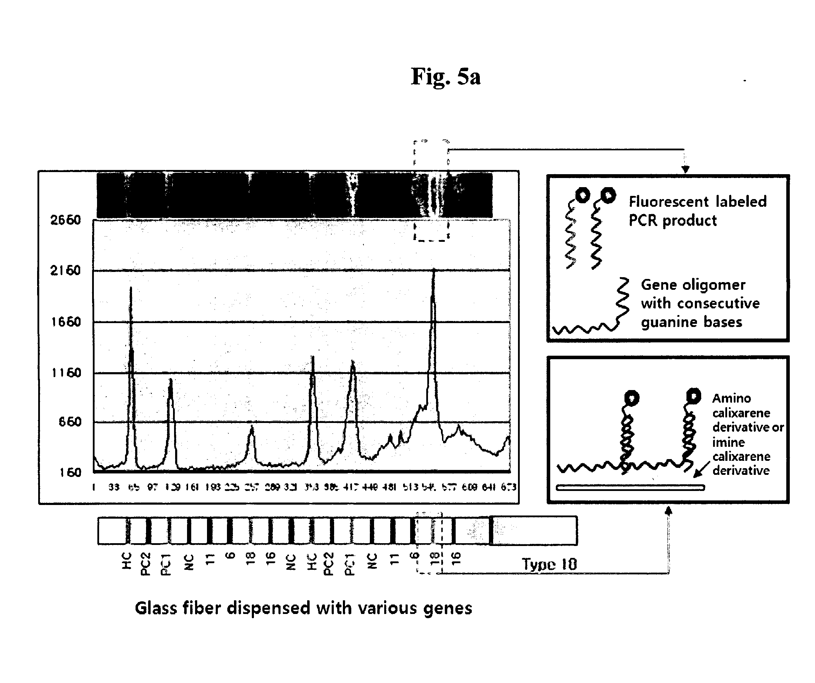

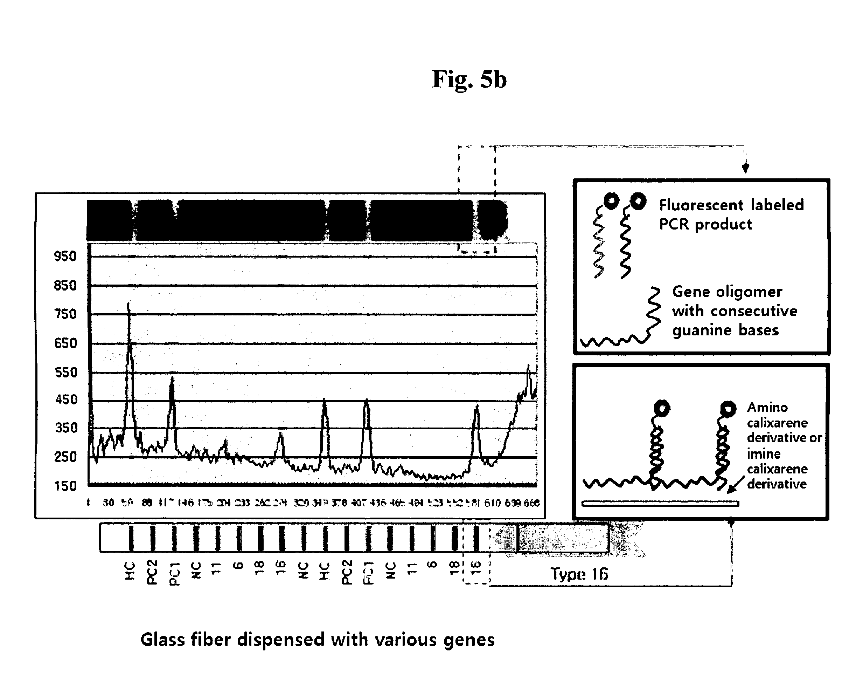

Fig. 1a is a diagram showing the process of preparing a monolayer by binding the derivative of Formula 1 on the surface of amine- modified glass fiber.
Fig. 1b is a diagram showing the process of preparing a monolayer by binding the derivative of Formula 2 on the surface of amine- modified glass fiber.

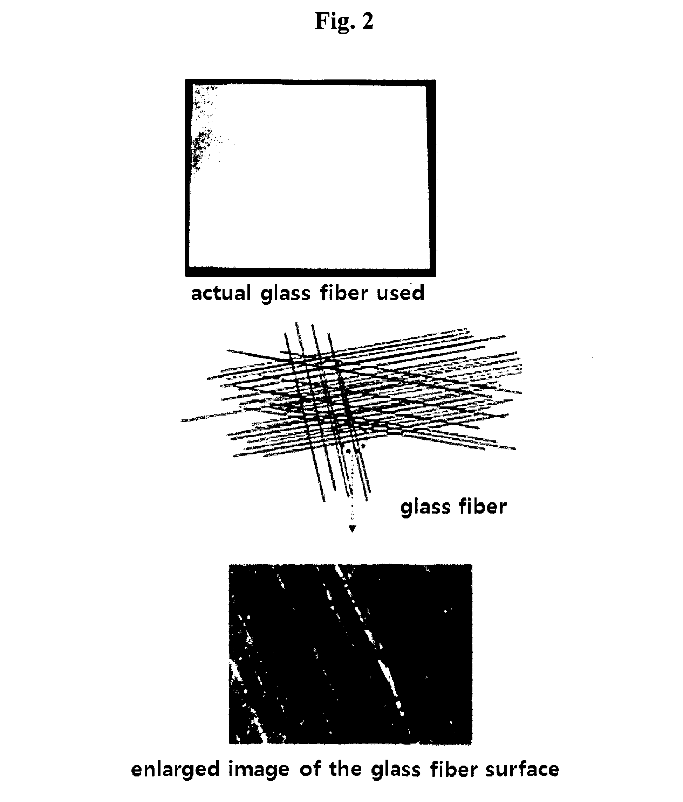

Fig. 2 is an image of the glass fiber, its enlarged image and a diagram. Thin glass fibers with diameters ranging from several nm to several hundred µm were weaved into a membrane structure. The glass fibers used in the present invention are produced by Millipore Co. of U.S.A. or Whatman Co. of U.K. The surface of these commercially available glass fibers produced from pure glass material were modified with calixarene derivate as shown in Formulae 1-3, with the same method used for the glass slides, and the glass fiber immobilized with probes were produced.
Fig. 3 is a diagram showing the process of preparing strip type DNA chip. The probe DNA spontaneously hybridizes on the glass fiber modified into macromolecular monolayer. The HPV genotyping oligonucleotides having consecutive guanines dissolved in liquid solvent were dispensed as separate lines on the glass fiber.
Fig. 4 is a diagram showing the hybridization reaction by applying the fluorescent labeled PCR product sample on the strip type DNA chip, where the sample spontaneously develops and hybridizes with the immobilized probe that is complementary to the genotype.
Fgs. 5a-5b are diagrams showing the results after applying the fluorescent labeled PCR products (Fig. 5a: HPV type 18, Fig. 5b: HPV type 16) on the glass fiber where HPV genotyping probes are dispensed and immobilized on the surface as lines. When the fluorescent labeled PCR product sample is applied at one end of the glass fiber, the sample spontaneously develops and hybridizes with the immobilized probe that is complementary to the genotype. Strong fluorescence signal is detected at locations where the base sequences of the PCR product match with the immobilized probe, whereas no fluorescence is detected at locations where no matching occurs. Therefore, the strip type DNA chip of this invention can be used for detecting and genotyping HPV. In the graph, y axis represents the arbitrary unit (AU) and x axis represents the data point (first dimensional location on the strip).

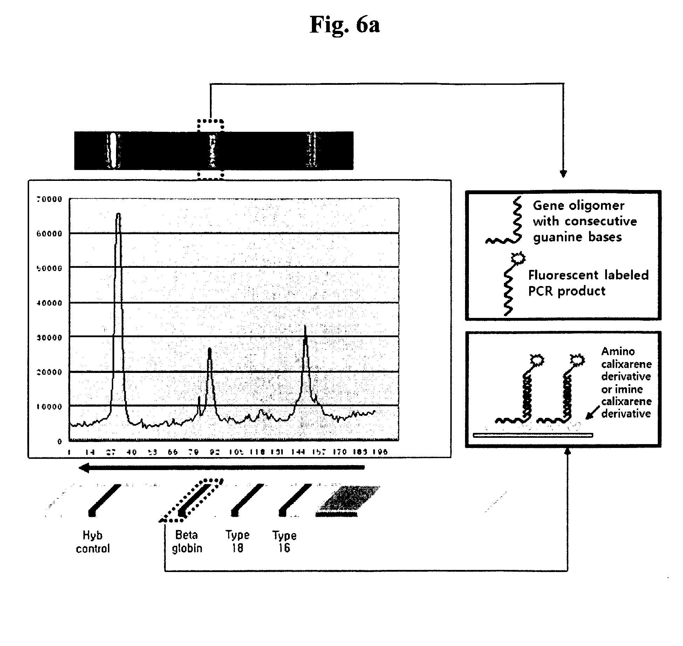

Figs. 6a-6b are diagrams showing the detection of HPV and beta-globin gene in test samples. The fluorescent intensity from the samples is normalized against the fluorescent intensity of beta-globin, the housekeeping gene in human cell. In the graph, y axis represents the arbitrary unit (AU) and x axis represents the data point (first dimensional location on the strip).

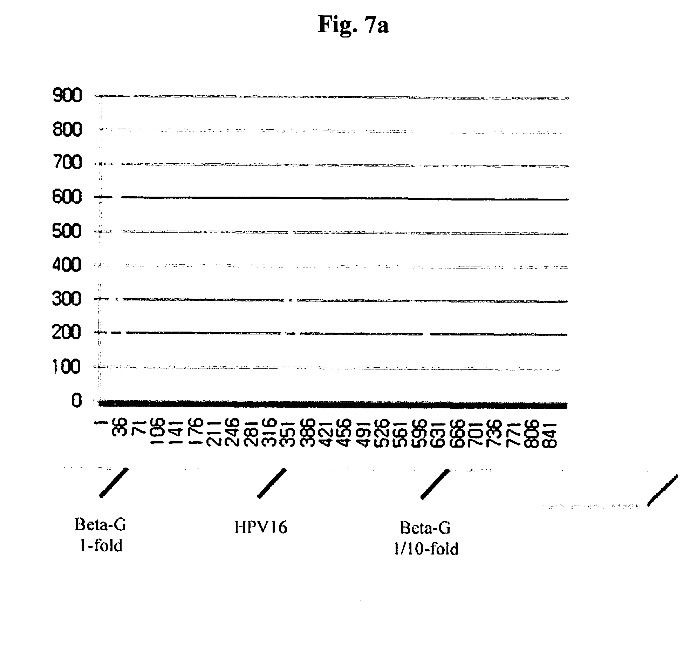

Figs. 7a-7c are graphs showing the quantitative analysis of HPV type 16 in the test sample. The fluorescent intensity from samples is normalized against the fluorescent intensity of beta-globin, the housekeeping gene in human cell. Fig. 7a-7c represent the analysis result of the PCR product using template DNA having 2.5 × 10⁴, 2.5 × 10³ and 0 copy numbers. In the graphs, y axis represents the arbitrary unit (AU) and x axis represents the data point (first dimensional location on the strip). In the lower graph of Fg.7c, y axis represents the arbitrary unit (AU) and x axis represents the copy number of the DNA template.

The present invention will now be described in further detail by examples. It would be obvious to those skilled in the art that these examples are intended to be more concretely illustrative and the scope of the present invention as set forth in the appended claims is not limited to or by the examples.

### EXAMPLES

### Example 1: Surface modification of the amine-modfied glass fiber using 5,II,23-tetradibenzylaminocalix[4]arene-I,3-hexanealdehyde (TDBACAHA)

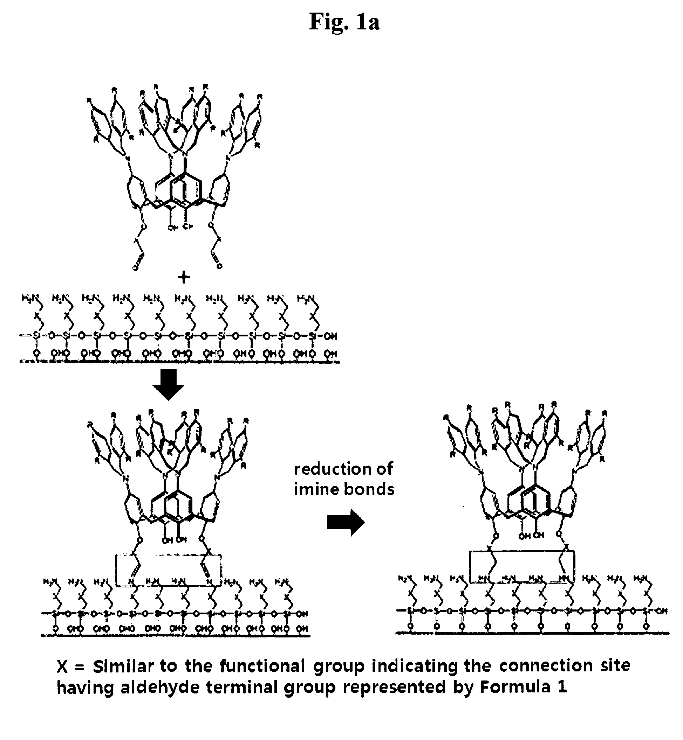

A solution was prepared by dissolving 0.1-0.5 mM of a derivative of Formula 1, 5,II,17,23-tetradibenzylaminocalix[4]arene-I,3-hexaneaidehyde (TDBACAHA, Korean Pat. No. 698763), in an organic solvent such as CHCl₃. According to the method shown in Fig. 1a (Langmuir, 12:5338-5342(1996)), the glass fiber surface was modified to an amine group then immersed in the prepared solution for 1-24 hrs and washed with chloroform, acetone and water, respectively before drying. The glass fiber modified to aminocalixarene monolayer that will be used as DNA immobilization strip is shown in Fg. 1a.

### Example 2: Surface modification of the amine-modified glass fiber using 5, II, 17, 23-tetrabenzytimineaicoxycalix[4]arenedihexaldehyde (TBICAHA).

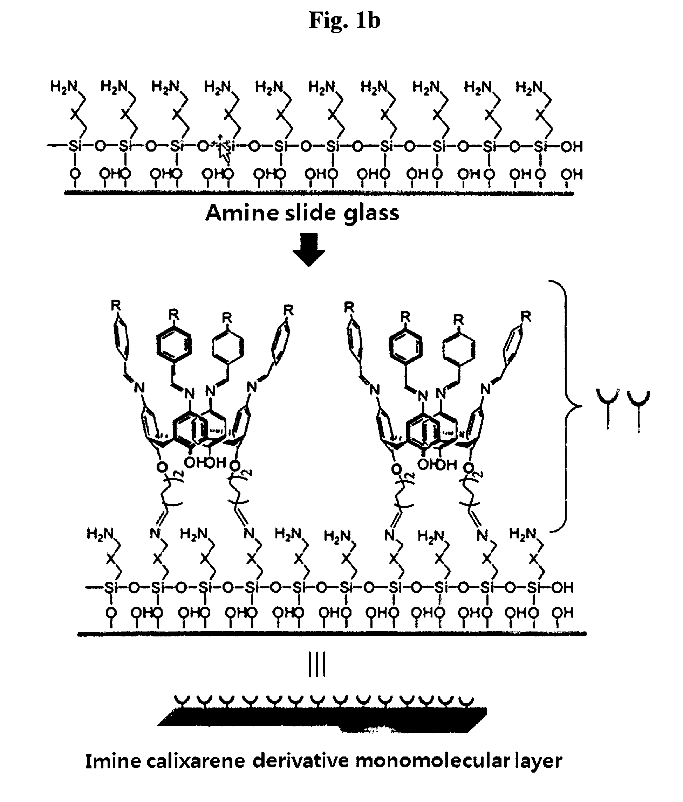

A solution was prepared by dissolving 0.1-0.5 mM of a derivative of Formula 2, 5,II,17,23-tetrabenzyliminealoocycalix[4]arenedihexaldehyde(TBICAHA, Korean Pat. No. 748082), in an organic solvent such as CHCl₃. According to the method shown in Fg. 1b (Langmuir, 12:5338-5342(1996)), the glass fiber surface was modified to an amine group then immersed in the prepared solution for 1-24 hrs and washed with chloroform, acetone and water, respectively before drying. The glass fiber modified to iminecalixarene monolayer that will be used as DNA immobilization strip is shown in Fig. 1 b.

### Example 3: HPV genotyping by hybridization with probe immobilized on the modified glass fiber substrate and HPV PCR product

### (a) Preparation of HPV DNA samples for PCR amplification

Followings are the *E. coli* strains carrying different types of HPV plasmid DNAs that are used in the Example: HPV-16 (ATCC 45113), HPV-18 (ATCC 45152), HPV-6b (ATCC 45150), HPV-11 (ATCC 45151).

Each of the *E. coli* strains carrying different type of HPV plasmid DNA were grown for 16hrs in LB media at 37°C using a shaking incubator. Plasmid DNA was extracted using "Accuprep Nano-Plus Plasmid Mini Extraction Kit (Cat. No. K-3111, Bioneer Co. Ltd. Republic of Korea), and adjusted to 10 ng/µl concentration to be used as control template DNA solution.

The purified HPV type DNA was amplified by PCR using primers shown in Table 1. The PCR primers used in the reaction were synthesized by Bioneer Co. Ltd. PCR reaction was carried out in a reaction solution purchased from Bioneer Co. Ltd., which includes 10 µl of PCR buffer, 1.5 mM MgCl₂, 250 µM dNTP, 30 mM KCl, 10 mM Tris- HCl (pH9.0), Taq polymerase (1 unit) and 1 µl of primer (10 pmol/ µl), 7 µl of distilled water, and 1 µl of template DNA. The PCR reaction cycle was 5 min at 94°C, 35 cycles of denaturation for 1 min at 94°C, 45 sec at 45°C and 1 min at 72°C followed by 5min at 72°C. Five µl of the reaction solution and a DNA size standard maker was electrophoresised on 2% agarose gel. DNA bands in each lane were stained with 0.00005% ethidium bromide solution and visualized under a UV light

**Table 1**

| Primer name | Base sequence (5'→3') |
|---|---|
| Forward direction | GATGGTGATATGGrAGATACAGGATTT |
| Cy5-Reverse direction | CY5-CCTAGTGGCTCTATGGTAACCTCTGACGC |

| | |
|---|---|
| *Cy-5 was purchased from Telechem. U.S.A | |

### (b) Immobilization of the probe on the glass fiber

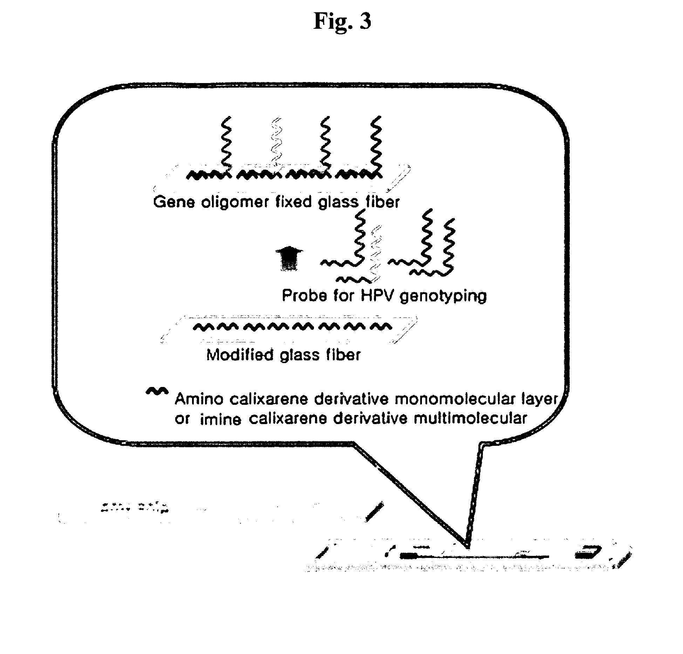

A Biodot dispenser (Model No. XYZ 3050, U.S.A) was used to immobilize the probe DNA (Fig. 3). HPV-16, -18, -6, -11, positive control 1 (PC-1), positive control 2 (PC-2), negative control (NC) and hybridization control (HC) each containing 9 consecutive guanines (shown in Table 2) were dissolved in BMT dispensing solution (Biomatrix Technology Co. Republic of Korea) at a concentration of 7-30 pmo)/µl to prepare the immobilization solution (100 mM ammonium ion). The immobilized solution was dispensed at the rate of 20 mm/sec (0.7 µl/Cm) on the modified glass fiber (Biomatrix Technology Co. Republic of Korea) using a dispensing nozzle. The probe was applied on the glass fiber as a linear band with a width of 0.2-3.0 mm and left to immobilize spontaneously. After immobilizing at room temperature for 1-24 hrs, the glass fiber was washed and immersed in 250 ml of BMT blocking solution (4xSSC, 1% casein and 0.5% polyethyleneglycol) for 10-30 min to block the remaining surface where oligo-DNAs did not immobilized. Fig. 5 shows the manufactured strip after drying the incubator at 40-50°C for 0.5-4 hrs.

**Table 2**

| Probe | Base sequence (5'→3') | HPV type |
|---|---|---|
| HPV-1 | ggg ggg ggg t tca aat tat ttt cct aca | HPV-16 |
| HPV-2 | ggg ggg ggg gt gtg tat tct ccc tct | HPV-18 |
| HPV-3 | ggg ggg ggg agt ata tat gtt aac acc | HPV-6 |
| HPV-4 | ggg ggg ggg tct gta gct act agt att tat gta cat aca | HPV-11 |
| PC-1^{*} | ggg ggg ggg tat ttt cct aca cct agt gg | Positive control |
| | ggg ggg ggg tat tct ccc tct cct agt gg | |
| PC-2^{**} | ggg ggg ggg tat gtt aac acc cct agt gg | Positive control |
| | ggg ggg ggg tat gta cat aca cct agt gg | |
| NC^{***} | ggg ggg ggg aaa gct gct gct cgt cgt cgt cgt | Negative control |
| HC^{****} | ggg ggg ggg ttt aca cct agt ggc tct atg gtg tcc tct | Hybridization control |

| | | |
|---|---|---|
| ^{*} two types of probes were mixed and coated (positive control group for HPV -16, -18) ^{**} two types of probes were mixed and coated (positive control group for HPV -6, -11) ^{***} Probe that binds to the nudeotide sequence that does not exist in HPV sequence ^{****}Probe that hybridizes with Cy-5 reverse primer in Table 1 | | |

### (c) Method for detecting fluorescent labeled PCR product hybridized with probe immobilized on a glass fiber

The surface of glass fiber substrate was modified following Example 1, and the probe was immobilized on the glass fiber according to Example 3-(b). A case was assembled as shown in Fig. 3 to detect a specific DNA from the PCR products that flow across the glass fiber. Developing solution was prepared by mixing 5 µl of PCR product from Example 3-(a) and 75 µl of BMT hyb-mix A (6xSSC, 20% formamide and 0.05% triton X-100) in a 1.5 ml tube. The mixed solution was heated for 3 min at 100°C and left to cool for 3 min on ice. Eighty µl of the mixed solution was injected into the inlet of the assembled strip. The strip was left to stand at room temperature for 3-120 min for the mixed solution to develop and hybridize. After hybridization, 100 µl of BMT Wa-B-2 solution (4x SSC, Biomatrix Technology Co. Republic of Korea) was injected and the strip was detached after 5 min. The reaction terminated glass fiber (strip) was mounted on a slide for a quantitative analysis of the fluorescent intensity using microarray scanner (GSI Lumonics, U.S.A). The analysis results are shown as a strip on top panel of Fig. 5a and Fig. 5b. The graphs in Fig. 5a and 5b show the results from BMT HPV strip reader Type-1 (Biomatrix Technology Co. Republic of Korea) that was used for analyzing the strips. The fluorescent intensity ratio of HC, PC-1 and PC-2 and that of HPV type were highly correlative. PC-1 and PC-2 were positive controls to confirm whether HPV virus existed or not.

As shown dearly in Fig. 5a-5b, the samples contacting HPV types showed specific hybridization reaction occurring at the bands (line) where HPV type specific probe was immobilized. Hybridization reaction in the PC region confirmed that the virus existed and no hybridization reaction was detected in the region where different types of probe were used. Therefore, present invention describes the development of a strip type DNA chip will enable HPV genotype analysis due to its high selectivity and less nonspecific binding.

### Example 4: Detection of both HPV and beta-globin

### (a) Immobilization of oligoprobe for DNA analysis using Beta-globin gene PCR product

A Biodot dispenser (Model No. XYZ 3050, U.S.A) was used to immobilize probe on the glass fiber-based strip. Oligoprobes complementary to the PCR product of HPV type 16, HPV-type 18, beta-globin and hybridization control (HC) all containing 9 consecutive guanines (shown in Table 3) were dissolved in BMT dispensing solution (Biomatrix Technology Co. Republic of Korea) at a concentration of 7-30 pmol/µl to prepare the immobilization solution. The immobilized solution was dispensed at the rate of 20 mm/sec (0.7 µl/Cm) on the glass fiber prepared according to Example 1 or Example 2. The probe was applied on the glass fiber as a linear band with a width of 0.1-3.0 mm. After immobilizing at room temperature for 1-24 hrs, the glass fiber was immersed in 250 ml of BMT blocking solution (4xSSC, 1% casein and 0.5% polyethyleneglycol) for 10-30 min to block the remaining surface where oligo-DNAs did not immobilized, then strip was manufacture by drying in the incubator at 40-50°C for 0.5-4 hrs.

**Table 3**

| Probe | Base sequence (5'→3') | HPV type |
|---|---|---|
| HPV-1 | ggg ggg ggg t tca aat tat tat cct aca | HPV-16 |
| HPV-2 | ggg ggg ggg gt gtg tat tct ccc tct | HPV-18 |
| Beta-G | ggg ggg ggg c tgc cct gtg ggg caa g | Beta-globin |
| HC^{*} | ggg ggg ggg ttt aca cct agt ggc tct atg gtg tcc tct | Hybridization control |

| | | |
|---|---|---|
| ^{*}Probe that hybridizes with Cy-5 reverse primer | | |

### (b) PCR amplification of the target DNA

Cells collected from uterine cervix using cytobrush were immersed in a 15ml tube containing 5 ml PBS then vortexed for 2 min to separate the cells from the cytobrush. The solution was centrifuged for 10 min at 3,000g and the supernatant was removed carefully from the cell pellets. The cell was extracted using "Accuprep Nano-Plus Plasmid Mini Extraction Kit (Cat. No. K-3111, Bioneer Co. Ltd. Republic of Korea) and adjusted to 10 ng/µl concentration to be used as control template DNA solution.

The purified HPV type DNA was amplified by PCR using primers shown in Table 4. The PCR primers were synthesized by Bioneer Co. Ltd. PCR reaction was carried out in a reaction solution purchased from Bioneer Co. Ltd., which includes 10 µl of PCR buffer, 1.5 mM MgCl₂, 250 µM dNTP, 30 mM KCl, 10 mM Tris- HCl (pH9.0), Taq polymerase (1 unit) and 1 µl of primer (10 pmol/ µl), 7 µl of distilled water, and 1 µl of template DNA. The PCR reaction cycle was 5 min at 94°C, 35 cycles of denaturation for 1 min at 94°C, 45 sec at 45°C and 1 min at 72°C followed by 5 min at 72°C. Five µl of the reaction solution and a DNA size standard maker was electrophoresised on 2% agarose gel. DNA bands in each lane were stained with 0.00005% ethidium bromide solution and visualized under a UV light

**Table 4**

| Primer Name | Base sequence (5'→3') |
|---|---|
| Forward direction | caa ctt cat cca cgt tca cc |
| CyS-Reverse direction | CY5-gg tga acg tgg atg aag ttg |

### (c) Fluorescent detection method by hybridizing fluorescence labeled PCR product and beta-globin probe immobilized on a glass fiber

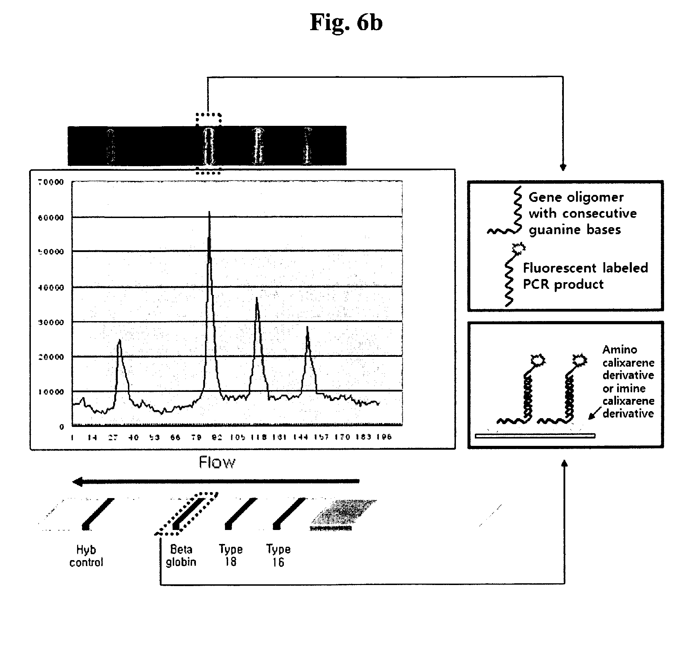

The glass fiber substrate immobilized with the probe was assembled into a strip shown in Fig. 3. Specific DNA and beta-globin in the sample solution was identified by the binding of immobilized probe and the gene in the sample solution that flows across the glass fiber. For the hybridization reaction between the PCR amplified product and the probe, 5 µl of PCR product of HPV virus prepared according to Example 3-(a), 5 µl of PCR product of beta-globin prepared according to Example 4-(b) and 75 µl of BMT hyb-mix A (6xSSC, 20% formamide and 0.05% triton X-100) were mixed to make 85 µl of hybridization reaction solution in a 1.5 ml tube. The solution was heated for 3 min and left to cool for 3 min on ice. Eighty five µl of the mixed solution was injected into the inlet of the assembled strip. The strip was left to stand at room temperature for 3-120 min for the hybridization reaction solution to develop and hybridize. After hybridization, 100 µl of BMT Wa-B-2 solution (4x SSC, Biomatrix Technology Co. Republic of Korea) was injected to develop the wash solution, then the strip was analyzed using BMT HPV strip reader Type-1 (Biomatrix Technology Co. Republic of Korea, Fig. 6a and 6b). The glass fiber (strip) was detached from the case and mounted on a slide for a quantitative analysis of the fluorescent intensity using microarray scanner (GSI Lumonics, U.S.A). Based on the results from both analyses, the fluorescent intensity ratio obtained from HC, beta-globin and HPV genotypes are highly correlative to the result collected form scanner and BMT reader, respectively.

### Example 5: Quantification of HPV using beta-globin gene and hybridization control (HC)

### (a) Immobilization of oligoprobe for DNA analysis using PCR product of beta-giobin gene

A Biodot dispenser (Model No. XYZ 3050, U.S.A) was used to immobilize probe on the glass fiber-based strip. Oligoprobes complementary to the PCR product of HPV type 16 and beta-globin containing 9 repeated guanine bases (shown in Table 3) that can hybridize at 1-fold and 1/10-fold diluted concentration were dissolved in BMT dispensing solution (Biomatrix Technology Co. Republic of Korea) at a concentration of 7-30 pmol/µl to prepare the immobilization solution. The immobilized solution was dispensed at the rate of 20 mm/sec (0.7 µl/Cm) on the glass fiber prepared according to Example 1 or Example 2. The probe was applied on the glass fiber as a single band with a width of 0.1-3.0 mm. After immobilizing at room temperature for 1-24 hrs, the glass fiber was immersed in 250 ml of BMT blocking solution (4xSSC, 1% casein and 0.5% polyethyleneglycol) for 10-30 min to block the remaining surface where oligo-DNAs did not immobilized, then incubated at 40-50°C for 0.5-4 hrs to dry.

**Table 5**

| Probe | Base sequence (5'→3') | HPV type |
|---|---|---|
| HPV-1 | ggg ggg ggg t tca aat tat ttt oct aca | HPV-16 |
| Beta-G | ggg ggg ggg c tgc oct gtg ggg caa g | Beta-globin |

### (b) PCR amplification of the target sequence

Cells collected from uterine cervix using cytobrush were immersed in a 15ml tube containing 5 ml PBS and vortexed for 2 min to separate the cells from the cytobrush. The solution was centrifuged for 10 min at 3,000g and the supernatant was removed from the cell pellets. The cell was extracted using "Accuprep Nano-Plus Plasmid Mini Extraction Kit (Cat. No. K-3111, Bioneer Co. Ltd. Republic of Korea) and adjusted to 10 ng/µl concentration. This was further prepared at a concentration of 2.5 x 10⁴ and 2.5 x 10³ copies and used as control template DNA solution.

The PCR was performed for HPV type 16 and beta-globin DNA using primers shown in Table 6 and using the purified DNA as template. The PCR primers used in this experiment was ordered and purchased from Bioneer Co. Ltd. PCR reaction was carried out in a reaction solution purchased from Bioneer Co. Ltd., which includes 10 µl of PCR buffer, 1.5 mM MgCl₂, 250 µM dNTP, 30 mM KCl, 10 mM Tris- HCl (pH9.0), Taq polymerase (1 unit) and 1 µl of primer (10 pmol/ µl), 7 µl of distilled water, and 1 µl of template DNA. The PCR reaction cycle was 5 min at 94°C, 35 cycles of denaturation for 1 min at 94°C, 45 sec at 45°C and 1 min at 72°C followed by 5 min at 72°C. Five µl of the reaction solution and a DNA size standard maker was electrophoresised on 2% agarose gel. DNA bands in each lane were stained with 0.00005% ethidium bromide solution and visualized under a UV light.

**Table 6**

| Primer name | Base sequence (5'→3') |
|---|---|
| HPV (Forward direction) | gat ggt gat atg gta gat aca gga ttt |
| HPV (Reverse direction) | CY5-cct agt ggc tct atg gta acc tct gac gc |
| Beta-G (Forward direction) | caa ctt cat cca cgt tca oc |
| Beta-G (Reverse direction) | CY5-gg tga acg tgg atg aag ttg |

### (c) Fluorescent detection method by hybridizing fluorescence labeled PCR product and beta-globin probe immobilized on a glass fiber

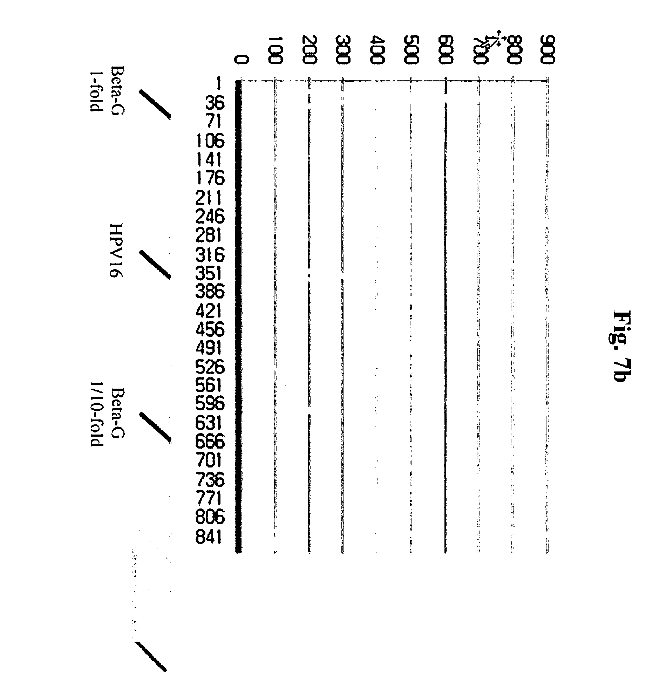

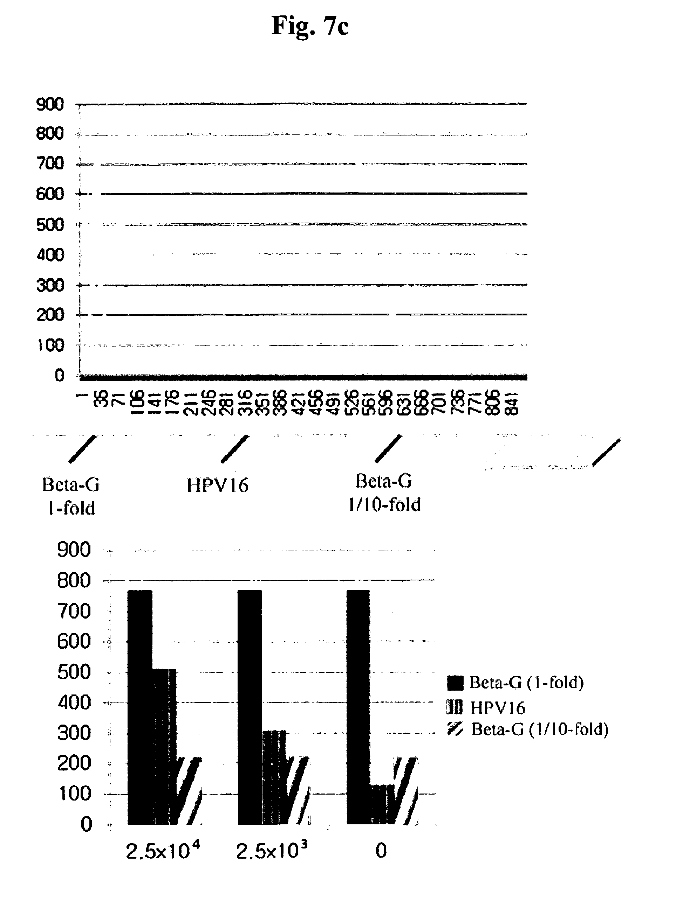

The glass fiber substrate immobilized with the probe was assembled into a strip shown in Fig. 3. Specific DNA and beta-globin in the PCR product was identified by the binding of immobilized probe and DNA from the solution that flows across the glass fiber. The expression level of HPV gene at specific concentration was quantified by applying the PCR product form different concentrations of template DNA, 2.5 x 10⁴ and 2.5 x 10³ copies, on the strip and quantifying the fluorescence intensity relative to hybridization control gene levels (HC; HC probe from Table 2) and beta-globin. For the hybridization reaction between fluorescent labeled PCR product and the probe, 5 µl each from PCR reaction mixture of HPV virus and beta-globin, prepared as in Example 5-(b) and 75 µl of BMT hyb-mix A (6xSSC, 20% formamide and 0.05% triton X-100) were added to make total of 85 µl hybridization solution in 1.5 ml tube. This solution was heated for 3 min in water at 100°C and left to cool for 3 min on ice. Eighty five µl of the mixed solution was injected into the inlet of the assembled strip. The strip was left to stand at room temperature for 3-120 min for the hybridization reaction solution to develop and hybridize. After hybridization, 100 µl of BMT Wa-B-2 solution (4x SSC, Biomatrix Technology Co. Republic of Korea) was injected to develop the wash solution, then BMT HPV strip reader Type-1 (Biomatrix Technology Co. Republic of Korea) was used to analyze the strip (Fig. 7a, 7b and 7c). The current invention confirms that the amount of HPV genotypes can be quantified using fluorescent intensity of the beta-globin, that is by analyzing the fluorescent ratio between high and low levels of beta-globin and HPV genotypes detected from reaction terminated glass fiber (strip) using the BMT reader.

Having described a preferred embodiment of the present invention, it is to be understood that variants and modifications thereof falling within the spirit of the invention may become apparent to those skilled in this art, and the scope of this invention is to be determined by appended claims and their equivalents.

## Claims

1. A DNA chip for detecting or genotyping HPV, which comprises (a) a calixarene derivative coated on the surface of a solid substrate; and (b) a probe hybridizable with a nucleotide sequence of HPV (human papilloma virus) immobilized on the surface of the solid substrate, wherein the probe comprises (i) a hybridizing portion hybridizable with the nucleotide sequence of HPV and (ii) an immobilization portion having at least two guanine bases.

2. The DNA chip according to daim 1, wherein the calixarene derivative is represented by the following Formula 1, Formula 2 or Formula 3: 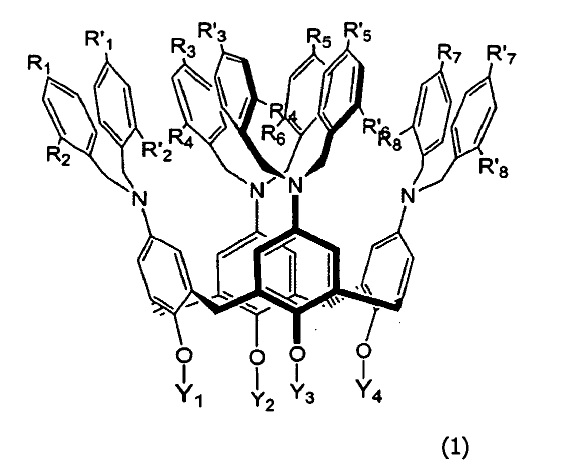 wherein, R₁, R₂, R₃, R₄, R₅, R₆, R₇, R₈, R'₁ ,R'₂ ,R'₃ ,R' ₄ ,R' ₅ ,R' ₆ ,R '₇and R'₈ independently represent -H, -CH₃, -C₂H₅, -C₃H₇, -OCH₃, - Cl, -C₆H₅, -OH, -OCH₂CH₃, -Br, -CF₃, -OCH₂C₆H₅ - OC₆H₅, -OC₆H₄CH₃, -OC₆H₄C(CH₃)₃, -OC₆H₄CF₃, -C₆H₄Cl, -OCOCH₃, -NHCOCH₃, -CONHCH₃, - CN, -COOH or -COOR; -COOR; R in -COOR is -CH₃ or -C₂H₅; Y₁, Y₂, Y₃ and Y₄ independently represent -H, - (CH₂)ₙ -CH=O, -(CH₂)ₙ-SH, -(CH₂CH₂O)ₘ-CH₂CH₂-CH=O, -(CH₂CH₂O)ₘ-CH₂CH₂-SH, -(CH₂)ₘ-C₆H₄-(CH₂)_{c}-Z or -CO-(CH₂)ₘ₋₁-C₆H₄-(CH₂)_{c}-Z; Z represents -SH, -CHO, -COOH or - NH₂; -C₆H₄- and C₆H₅ represent a phenyl group; n is an integer of 2-15, m is an integer of 1-10, and c is an integer of 0-10; 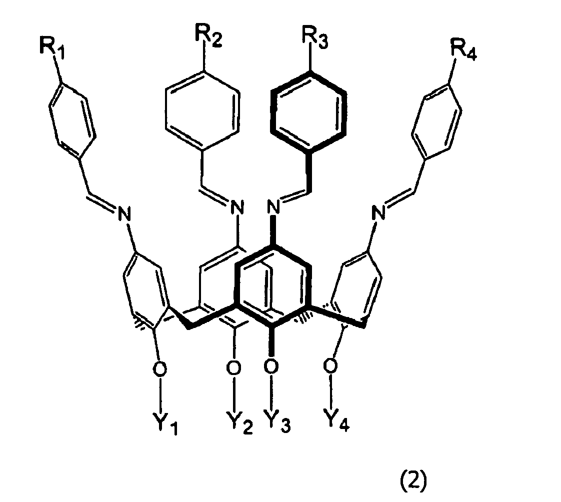 wherein, R₁, R₂, R₃ and R₄ independently represents -H, -CH₃, -C₂H₅, -C₃H₇, -OCH₃, - Cl, -C₆H₅, -OH, -OCH₂CH₃, -Br, -CF₃, -OCH₂C₆H₅ -OC₆H₅, -OC₆H4CH₃, -OC₆H₄C(CH₃)₃, - OC₆H₄CF₃, -C₆H₄Cl, -OCOCH₃, -NHCOCH₃, -CONHCH₃, -CN, -COOH or -COOR; -COOR; R in - COOR is -CH₃ or -C₂H₅; Y₁, Y₂, Y₃ and Y₄ independently represent -H, - (CH₂)ₙ -CH=O, -(CH₂)ₙ-SH, -(CH₂CH₂O)ₘ-CH₂CH₂-CH=O, -(CH₂CH₂O)ₘ-CH₂CH₂-SH, -(CH₂)ₘ-C₆H₄-(CH₂)_{c}-Z or -CO-(CH₂)ₘ₋₁-C₆H₄-(CH₂)_{c}-Z; Z represents -SH, -CHO, -COOH or -NH₂; -C₆H₄- and C₆H₅ represent phenyl groups; n is an integer of 2-15, m is an integer of 1-10, and c is an integer of 0-10; 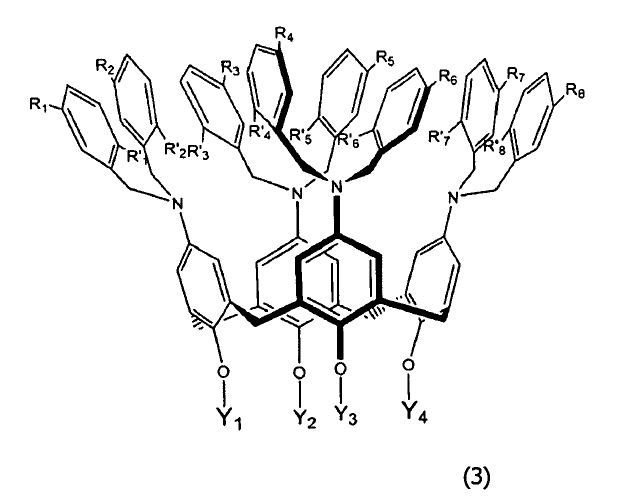 wherein, R₁, R₂, R₃, R₄, R₅, R ₆, R₇, R₈. R'₁ ,R'₂ ,R'₃ ,R' ₄ ,R' ₅ ,R' ₆ ,R '₇ and R'₈ independently represent -H, -CH₃, -C₂H₅, -C₃H₇, -OCH₃, - Cl, -C₆H₅, -OH, -OCH₂CH₃, -Br, -CF₃, -OCH₂C₆H₅ -OC₆H₅, -OC₆H₄CH₃, -OC₆H₄C(CH₃)₃, -OC₆H₄CF₃, -C₆H₄Cl, -OCOCH₃, -NHCOCH₃, - CONHCH₃, -CN, -COOH or -COOR; -COOR; R in -COOR is -CH₃ or -C₂H₅; Y₁, Y₂, Y₃ and Y₄ independently represent -H, - (CH₂)ₙ -CH=O, -(CH₂)ₙSH, -(CH₂CH₂O)ₘ-CH₂CH₂-CH=O, - (CH₂CH₂O)ₘ-CH₂CH₂-SH, -(CH₂)ₘ-C₆H₄-(CH₂)_{c}-Z or -CO-(CH₂)ₘ-C₆H₄-(CH₂)_{c}-Z; Z represents-SH, -CHO, -COOH or -NH₂; -C₆H₄- and C₆H₅ are phenyl groups; n is an integer of 2-15, m is an integer of 1-10, and c is an integer of 0-10; with an exception of the following compound: all of R₁, R₂, R₃, R₄, R₅, R₆, R₇, R₈, R'₁ ,R'₂ ,R'₃ ,R' ₄ ,R' ₅ ,R' ₆ ,R '₇and R'₈ are -H.

3. The DNA chip according to daim 1, wherein the probe hybridizable with the nucleotide sequence of HPV contains 7-15 guanine bases at its 5'- or 3'-end.

4. The DNA chip according to daim 1, wherein the solid substrate is selected from the group consisting of glass, glass fiber, metal, metal oxide, ceramic, quartz, silicon, semiconductor, Si/SiO₂, wafer, germanium, gallium arsenide, carbon, carbon nanotube, polymer, sepharose, agarose and colloid.

5. The DNA chip according to daim 4, wherein the solid substrate is the glass fiber.

6. The DNA chip according to daim 1, wherein the DNA chip comprises probes hybridizable with nucleotide sequences of at least two HPV types selected from the group consisting of HPV types 16, 18, 31, 33, 35, 39, 45, 51, 52, 56, 58, 59 and 68.

7. The DNA chip according to daim 1, wherein the DNA chip further comprises a probe hybridizable with a housekeeping gene in a human cell.

8. The DNA chip according to daim 7, wherein the housekeeping gene is GAPDH (glyceraldehydes 3-phosphate dehyrogenase) gene, β-actin gene, PKG1 (phosphoglycerate kinase 1) gene, β-globin gene or 28S RNA gene.

9. The DNA chip according to daim 1, wherein the DNA chip is in the form of a membrane strip; at least two probes hybridizable with HPV types are immobilized on specific areas along the membrane strip; a DNA sample to be analyzed is applied at one end of the membrane strip and allowed to flow on the membrane strip surface.

10. The DNA chip according to daim 1, wherein the DNA chip further comprises a hybridization control probe hybridizable with a primer to amplify the nudeotide sequence of HPV in an analysis sample.

11. The DNA chip according to daim 1, wherein the DNA chip further comprises a positive control probe hybridizable with a HPV type-nonspedfic nudeotide sequence.

12. The DNA chip according to daim 1, wherein the DNA chip further comprises a negative control probe being not hybridizable with the nucleotide sequence of HPV.

13. The DNA chip according to daim 9, wherein the DNA chip comprises (i) the membrane strip containing a glass fiber as the solid substrate, (ii) at least two probes spedfically hybridizable with nudeotide sequences of at least two HPV types selected from the group consisting of HPV types 16, 18, 31, 33, 35, 39, 45, 51, 52, 56, 58, 59 and 68; each of the probes is immobilized on a specific area on the membrane strip, and (iii) a probe hybridizable with a housekeeping gene in a human cell.
